(19) Europäisches Patentamt
European Patent Office
Office européen des brevets

(11) **EP 3 079 661 B1**

(12) **EUROPÄISCHE PATENTSCHRIFT**

(45) Veröffentlichungstag und Bekanntmachung des Hinweises auf die Patenterteilung:
**05.05.2021 Patentblatt 2021/18**

(21) Anmeldenummer: **14811829.2**

(22) Anmeldetag: **08.12.2014**

(51) Int Cl.:
*A61K 9/20* *(2006.01)*     *A61K 9/50* *(2006.01)*
*A61K 31/00* *(2006.01)*    *A61K 31/485* *(2006.01)*
*A61P 1/10* *(2006.01)*     *A61P 25/36* *(2006.01)*

(86) Internationale Anmeldenummer:
**PCT/EP2014/076887**

(87) Internationale Veröffentlichungsnummer:
**WO 2015/086528 (18.06.2015 Gazette 2015/24)**

(54) **NALOXON-MONOPRÄPARAT UND MEHRSCHICHTTABLETTE**

NALOXONE MONO PREPARATION

MONO-PRÉPARATION DE NALOXONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.12.2013 EP 13005761**
**11.12.2013 EP 13005760**
**11.12.2013 EP 13005759**

(43) Veröffentlichungstag der Anmeldung:
**19.10.2016 Patentblatt 2016/42**

(73) Patentinhaber: **Algobate AG**
**6362 Stansstad (CH)**

(72) Erfinder:
- **REY, Hélène**
  **F-68128 Rosenau (FR)**
- **MUNDSZINGER, Olaf**
  **79588 Efringen-Kirchen (DE)**
- **GOLFIER, Isabelle**
  **F-68490 Bantzenheim (FR)**
- **JAKOB, Silvia**
  **79872 Bernau (DE)**

- **RUSCH, Oliver**
  **CH-4052 Basel (CH)**

(74) Vertreter: **Latscha Schöllhorn Partner AG**
**Grellingerstrasse 60**
**4052 Basel (CH)**

(56) Entgegenhaltungen:
**WO-A1-2009/085778     WO-A2-03/007802**
**DE-A1- 10 215 067     US-A- 4 987 136**

- **PROF DR I JURNA ET AL: "Retardiert freigesetztes Naloxon oral: Aufhebung der Obstipation durch orales Morphin ohne Beseitigung der Analgesie", SCHMERZ, SPRINGER VERLAG, BERLIN, DE, Bd. 7, Nr. 4, 1. Dezember 1993 (1993-12-01), Seiten 314-321, XP009176556, ISSN: 0932-433X**
- **L. LATASCH ET AL: "Aufhebung einer Morphin-induzierten Obstipation durch orales Naloxon", DER ANAESTHESIST, Bd. 46, Nr. 3, 24. März 1997 (1997-03-24), Seiten 191-194, XP055104659, ISSN: 0003-2417, DOI: 10.1007/s001010050390**

**Beschreibung**

**HINTERGRUND DER ERFINDUNG**

Die vorliegende Erfindung betrifft eine feste orale pharmazeutische Zusammensetzung, umfassend als einzigen Wirkstoff Naloxon oder ein pharmazeutisch annehmbares Salz davon, wobei die Zusammensetzung den Wirkstoff retardiert freisetzt.

Die vorliegende Erfindung betrifft auch eine feste orale pharmazeutische Zusammensetzung, umfassend als einzigen Wirkstoff Naloxon oder ein pharmazeutisch annehmbares Salz davon, zur Behandlung der Opioid-induzierten Obstipation.

Die vorliegende Erfindung betrifft eine Tablette, bevorzugt Mehrschichttablette, umfassend als Wirkstoffe einen Opioid-Agonisten oder ein pharmazeutisch annehmbares Salz davon sowie einen Opioid-Antagonisten oder ein pharmazeutisch annehmbares Salz davon, wobei die Tablette die Wirkstoffe retardiert freisetzt.

Die vorliegende Erfindung betrifft eine feste orale pharmazeutische Zusammensetzung, umfassend als einzigen Wirkstoff Naloxon oder ein pharmazeutisch annehmbares Salz davon, zur Verwendung in der Behandlung der Opioid-induzierten Obstipation.

Bei der Medikation von Schmerzmitteln vom Opioidtyp stellt die Obstipation eine bedeutende Nebenwirkung dar. Sie gilt als eine der häufigsten Nebenwirkungen und ist besonders in der Opioid-Dauertherapie eine unerwünschte Begleiterscheinung, die bei etwa 85 % der Patienten auftritt. Im Gegensatz zu anderen Opioid-induzierten Nebenwirkungen handelt es sich bei der Opioid-induzierten Obstipation um eine chronische Erscheinung, die im Laufe der Behandlung nicht an Intensität verliert. Die Wirkung der Opioide auf die Darmmotilität beruht vermutlich auf der Bindung der Opioide an Opioidrezeptoren des Gastrointestinaltraktes, die dort in verhältnismäßig großer Dichte anzutreffen sind.

Ziel einer entsprechenden Therapie ist es, diese periphere Nebenwirkung der Opioide aufzuheben, da die Opioid-induzierte Obstipation unangenehm und sehr schmerzhaft sein kann, häufig zum Abbruch der Opioid Therapie führt, und so den Behandlungserfolg der Opioide gefährdet. Da man bei der Opioid-induzierten Obstipation davon ausgeht, dass sie direkt und lokal über den gesamten Darm durch die Besetzung der Opioidrezeptoren bewirkt wird, sollte diese Nebenwirkung durch die Anwendung von Opioidantagonisten aufgehoben werden können. Die Verwendung von Opioidantagonisten ist allerdings nur dann sinnvoll, wenn sich die antagonistische Wirkung auf den Darm beschränkt und nicht die zentrale schmerzstillende Wirkung aufgehoben wird.

Ein geeigneter Opioidantagonist für die Behandlung der Opioid-induzierten Obstipation ist das Naloxon. Nach oraler Gabe wird Naloxon rasch und vollständig resorbiert und da die Substanz einem sehr ausgeprägten First-Pass-Metabolismus unterliegt, sind nur geringe Mengen des unveränderten Naloxons systemisch verfügbar. Der überwiegende Teil der applizierten Substanz liegt im Blut in Form von nicht oder nur schwach wirksamen Metaboliten wie etwas das Naloxon-3-Glukuronid oder das beta-6-Naloxol vor. Naloxon in geeigneter Dosis ist ein idealer Kandidat zur Behebung der Opioid-induzierten Obstipation: im Darm liegt es als aktive Substanz vor und kann so die lähmende Wirkung des Opioids auf den Gastrointestinaltrakt aufheben, während es nach der Resorption bei der ersten Leberpassage stark verstoffwechselt und so unwirksam wird. Somit wird die schmerzstillende Wirkung der Opioide nicht beeinflusst.

Die Opioid-induzierte Obstipation kann mit einer schnell freisetzenden Naloxon-Formulierung nicht erfolgreich behandelt werden, da die Lähmung den ganzen Gastrointestinaltrakt und nicht nur Duodenum und obere Teile des Dünndarms betrifft. WO 2011/117306 offenbart eine Zweischichttablette, die in der einen Schicht einen Opioid-Agonisten und in der anderen Schicht einen Opioid-Antagonisten enthält, wobei die Tablette beide Wirkstoffe schnell freisetzt. Vorteil dieser Zweischichttablette ist zwar die Unterdrückung von durch Opioid-Agonisten bewirkten Nebenwirkungen, hierbei steht aber die Opioid-induzierte Obstipation nicht im Vordergrund. Das im Markt befindliche Kombinationspräparat Targin®, in dem der Opioid-Agonist Oxycodon in Form des Hydrochloridsalzes und der Opioid-Antagonist Naloxon ebenfalls in Form seines Hydrochloridsalzes in Mischung vorliegen, setzt zwar die Wirkstoffe retardiert frei und ist somit zur parallelen Behandlung von Schmerz sowie Opioid-induzierter Obstipation geeignet. Diese monolithische Formulierung hat jedoch den Nachteil, dass die Freisetzungskinetik für beide Wirkstoffe nicht variable und daher der individuelle Behandlungserfolg nur schwer zu optimieren ist.

Weitere Präparate, welche Oxycodon und Naloxon umfassen sind bekannt. Diese sind dadurch gekennzeichnet, dass die Wirkstoffe aus dem Präparat verzögert, gleichbleibend und unabhängig voneinander freigesetzt werden. Auch bekannt sind feste orale pharmazeutische Zusammensetzungen, umfassend als einzigen Wirkstoff Naloxon oder ein pharmazeutisch annehmbares Salz davon, wobei die Zusammensetzung den Wirkstoff retardiert freisetzt. Das Freisetzungsprofil dieser Präparate bedarf einer erhöhten Retardierung, wobei sich die spezifische retardierten Freisetzung des Wirkstoffes entlang den gesamten Verdauungstrakt hinweg wirkt, eine Methode, um ein solches Freisetzungsprofil zu erreichen ist bisher nicht bekannt.

Neben Infusionslösungen zur Behandlung von Opioid-Vergiftungen sind im Markt nur Naloxon-Kombinationspräparate erhältlich, in welchen Naloxon und Opiat in einem festen Mengenverhältnis nebeneinander vorliegen. Zur Behandlung der Opioid-induzierte Obstipation wäre es aber wünschenswert, über ein Naloxon-Monopräparat zu verfügen, da dieses zum einen unabhängig von der Natur des Opiats und zum anderen in variabler Menge verabreicht werden kann, wodurch die gewünschte Naloxonmenge exakt appliziert und so ein optimaler Behandlungserfolg erreicht werden kann. Zwar sind in der Patentliteratur Naloxon-Monopräparate beschrieben, wie in WO 98/25613 A2. Diese Präparate setzen aber das Naloxon gezielt in Abhängigkeit vom Umgebungs-pH-Wert des Magen-Darm-Traktes frei, weshalb eine lokal gleichmäßige Versorgung über den gesamten Magen-Darm-Traktes hinweg mit Naloxon und somit ein optimaler Behandlungserfolg nicht möglich ist.

## GEGENSTAND DER ERFINDUNG

Aufgabe der vorliegenden Erfindung ist es eine feste orale pharmazeutische Zusammensetzung, umfassend als einzigen Wirkstoff Naloxon oder ein pharmazeutisch annehmbares Salz davon, wobei die Zusammensetzung den Wirkstoff retardiert freisetzt, bereitzustellen, die für eine zumindest zwölfstündige Gabe zur Behandlung der Opioid-indizierten Obstipation geeignet ist.

Diese Aufgabe wird gelöst durch eine feste orale pharmazeutische Zusammensetzung, umfassend als einzigen Wirkstoff Naloxon oder ein pharmazeutisch annehmbares Salz davon, wobei die Zusammensetzung den Wirkstoff retardiert freisetzt und eine in vitro-Freisetzungsrate des Wirkstoffs, gemessen unter Anwendung der Blattrührer-Methode gemäß Eur. Ph. bei 75 U/min in 500 ml 0,1 N Salzsäure bei 37 °C, von 0 % bis 75 % in 2 h, von 3 % bis 95 % in 4 h, von 20 % bis 100 % in 10 h, von 30 % bis 100 % in 16 h, von 50 % bis 100 % in 24 h und mehr als 80 % in 36 h aufweist.

Es wurde festgestellt, dass die erfindungsgemäße Zusammensetzung mit genanntem Freisetzungsprofil für eine zumindest zwölfstündige Gabe zur Behandlung der Opioid-indizierten Obstipation geeignet ist und entsprechend eine verhältnismäßig hohe Patientencompliance aufweist. Die in vitro-Freisetzungsrate wird unter Anwendung der Blattrührer-Apparatur (Apparatur 2) und der Blattrührer-Methode gemäß Eur.Ph. (Europäisches Arzneibuch, 7. Ausgabe, 3. Nachtrag, 2.9.3 "Wirkstofffreisetzung aus festen Arzneiformen", S. 5519-5526) bei 75 U/min in 500 ml 0,1 N Salzsäure bei 37 °C bestimmt. Die Menge an freigesetztem Wirkstoff wird vorzugsweise mittels UV-Detektion bei 220 nm bestimmt.

Die Opioid-induzierte Obstipation die mittels der erfindungsgemäßen Zusammensetzung behandelt kann, kann von sämtlichen Opioid-Analgetika oder Opioidanalogen-Analgetika, deren Salze sowie Mischungen davon hergerufen sein. Als Beispiele für entsprechende Analgetika seien genannt: Alfentanil, Allylprodin, Alphaprodin, Anileridin, Benzylmorphin, Bezitramid, Buprenorphin, Butorphanol, Clonitazen, Codein, Besomorphin, Dextromoramid, Dezocin, Diampromid, Diamorphon, Dihydrocodein, Dihydromorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Fentanyl, Heroin, Hydrocodon, Hydromorphon, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Narcein, Nicomorphin, Norlevorphanol, Normethadon, Nalorphin, Nalbuphen, Normorphin, Norpipanon, Opium, Oxycodon, Oxymorphon, Papaveretum, Pentazocin, Phenadoxon, Phenomorphan, Phenazocin, Phenoperidin, Piminodin, Piritramid, Propheptazin, Promedol, Properidin, Propoxyphen, Sufentanil, Tilidin, und Tramadol, wobei Hydrocodon, Morphin, Hydromorphon, Oxycodon, Buprenorphin, Codein, Fentanyl, Levorphanol, Meperidin, Methadon, Levomethadon und Dextromethadon erfindungsgemäß besonders bevorzugt sind.

Entsprechend einer bevorzugten Ausführungsform der erfindungsgemäßen Zusammensetzung ist es vorgesehen, dass die Zusammensetzung eine in vitro-Freisetzungsrate des Wirkstoffs von 0 % bis 50 % in 2 h, von 5 % bis 95 % in 4 h, von 20 % bis 90 % in 10 h, von mehr als 70 % in 18 h und von mehr als 80 % in 24 h aufweist.

Gemäß einer weiter bevorzugten Ausführungsform der erfindungsgemäßen Zusammensetzung ist es vorgesehen, dass die Zusammensetzung eine in vitro-Freisetzungsrate des Wirkstoffs von 0 % bis 38 % in 2 h, von 5 % bis 55 % in 4 h und von 20 % bis 75 % in 10 h aufweist.

Nach einer weiter bevorzugten Ausführungsform der erfindungsgemäßen Zusammensetzung ist es vorgesehen, dass die Zusammensetzung eine in vitro-Freisetzungsrate des Wirkstoffs von 0 % bis 50 % in 1 h, von 10 % bis 95 % in 4 h, von 35% bis 100% in 8 h, von 55% bis 100% in 12 h, von 70% bis 100 % in 16 h und von mehr als 90 % in 24 h aufweist.

Entsprechend einer weiter bevorzugten Ausführungsform der erfindungsgemäßen Zusammensetzung ist es vorgesehen, dass die Zusammensetzung eine in vitro-Freisetzungsrate des Wirkstoffs von 0 % bis 30 % in 1 h, von 0 % bis 40 % in 2 h, von 3 % bis 55 % in 4 h, von 10 % bis 65 % in 8 h, von 20 % bis 75 % in 12 h, von 30 % bis 88 % in 16 h, von 50 % bis 100 % in 24 h und von mehr als 80 % in 36 h aufweist.

Gemäß einer weiter bevorzugten Ausführungsform der erfindungsgemäßen Zusammensetzung ist es vorgesehen, dass die Zusammensetzung eine in vitro-Freisetzungsrate des Wirkstoffs von 10 % bis 30 % in 1 h, von 17 % bis 37 % in 2 h, von 27 % bis 47 % in 4 h, von 40 % bis 60 % in 8 h, von 50 % bis 70 % in 12 h, von 60 % bis 80 % in 16 h, von 80 % bis 100 % in 24 h aufweist.

Entsprechend einer besonders bevorzugten Ausführungsform der erfindungsgemäßen Zusammensetzung ist es vorgesehen, dass die Zusammensetzung den Wirkstoff unabhängig vom Umgebungs-pH-Wert des Magen-Darm-Traktes freisetzt. Dadurch wird gewährleistet, dass der gesamte Gastrointestinaltrakt unabhängig vom jeweiligen lokalen pH-Milieu gleichmäßig und kontinuierlich mit Naloxon bzw. einem Salz davon versorgt werden kann, wodurch eine weitere Optimierung des Behandlungserfolgs erzielbar ist. Die pH-unabhängige Wirkstofffreisetzung der erfindungsgemäßen Zusammensetzung kann durch Auswahl dem Fachmann bekannter pharmazeutischer Hilfsstoffe erhalten werden, wobei im Gastrointestinaltrakt lokale pH-Werte von etwa 1,2 (Magen) bis ca. 6,8 Kolon herrschen.

Unter einer vom Umgebungs-pH-Wert des Magen-Darm-Traktes unabhängigen Freisetzung des Wirkstoffs der erfindungsgemäßen Zusammensetzung wird dabei erfindungsgemäß bevorzugt verstanden, dass der Ähnlichkeitsfaktor f2 einer ersten in vitro-Freisetzung bei einem pH-Wert von 1,2 bis 6,8 und einer zweiten in vitro-Freisetzung bei einem beliebigen anderen pH-Wert von 1,2 bis 6,8 größer/gleich 50 ist.

Der Ähnlichkeitsfaktor f2 wird gemäß SHAH V.P., TSONG Y., SATHE P., & LIU J.P. (1998), "In vitro dissolution profile comparison-statistics and analysis of the similarity factor, f2", Pharmaceutical Research, 15, 889-896 bestimmt. Und zwar wird der Ähnlichkeitsfaktor f2 (engl. similarity factor) nach der folgenden Formel berechnet:

$$f_2 = 50 * log_{10}([1 + \frac{1}{n}\sum_{t=1}^{n}(R_t - T_t)^2]^{0,5} * 100)$$

In dieser Gleichung stellen Rt und Tt die freigesetzten Mengen des Wirkstoffs zum Zeitpunkt t beim ersten pH-Wert bzw. beim zweiten pH-Wert dar. n ist die Anzahl der Zeitpunkte. Der f2-Faktor wird unter den folgenden Bedingungen bestimmt: a) die minimale Anzahl an Zeitpunkten für eine Freisetzung beträgt 3 (der Zeitpunkt 0 ist ausgeschlossen), b) die Zeitpunkte für den ersten und zweiten pH-Wert sollten die Gleichen sein; c) für die freigesetzten Mengen für jeden Zeitpunkt wird für jeden pH-Wert ein Mittelwert von 12 Messungen angegeben, d) nicht mehr als ein Mittelwert gemessen oberhalb einer Freisetzung von 85 % darf in der Berechnung berücksichtigt werden, e) die relative Standartabweichung oder der Variationskoeffizient der Freisetzung bei einem pH-Wert sollte für den ersten Zeitpunkt kleiner als 20 % sein und für den zweiten und jeden weiteren Zeitpunkt kleiner als 10 %.

Für die f2-Faktor-Berechnung werden die in vitro-Freisetzungsprofile unter Anwendung der Blattrührer-Apparatur (Apparatur 2) und der Blattrührer-Methode gemäß Eur.Ph. (Europäisches Arzneibuch, 7. Ausgabe, 3. Nachtrag, 2.9.3 "Wirkstofffreisetzung aus festen Arzneiformen", S. 5519-5526) bei 75 U/min in 500 ml Puffer (gemäß Europäisches Arzneibuch, 7. Ausgabe, 7. Nachtrag, 4.1.3 "Pufferlösungen", S. 7671-7679) bei 37 °C bestimmt. Die Menge an freigesetztem Wirkstoff wird mittels UV-Detektion bei 220 nm bestimmt.

Gemäß einer weiter bevorzugten Ausführungsform der erfindungsgemäßen Zusammensetzung ist es vorgesehen, dass die Zusammensetzung eine Matrix umfasst, die den Wirkstoff retardiert freisetzt. Der Wirkstoff kann insbesondere dann kostengünstig retardiert freigesetzt werden, wenn er in einer retardiert freisetzenden Matrix enthalten ist.

Die erfindungsgemäße Zusammensetzung kann eine Matrix umfassen, die das Naloxon bzw. das pharmazeutisch annehmbare Salz davon retardiert freisetzt. Dabei kann es sich bei der Matrix erfindungsgemäß bevorzugt um eine sogenannte Gerüstmatrix, die quellbar oder nicht-quellbar sein kann, oder um eine sogenannte erodierende Matrix handeln. Die Matrix kann auch Eigenschaften von beiden, sowohl Gerüstmatrix als auch erodierende Matrix, aufweisen.

Bei einer Gerüstmatrix ist der Wirkstoff in einem Matrixgerüst inkorporiert. Während des Transportes der beladenen Gerüstmatrix durch den Gastrointestinaltrakt wird der Wirkstoff allmählich von den Verdauungssäften aus dem Matrixgerüst herausgelöst. Nachdem dies geschehen ist, wird das Matrixgerüst in mehr oder weniger unveränderter bzw. aufgequollener Form ausgeschieden. Bei einer erodierenden Matrix hingegen wird die Matrix abgebaut oder erodiert, wodurch Wirkstoffpartikel an die Oberfläche gelangen und sich auflösen. Dabei ist die Freisetzungsrate vom Ausmaß des Matrixabbaus bzw. deren Erosion abhängig.

Im Sinne der Ausbildung einer weitgehend stabilen Gerüstmatrix mit geeigneter Wirkstofffreisetzung ist es gemäß einer weiter bevorzugten Ausführungsform der erfindungsgemäßen Zusammensetzung vorgesehen, dass die Matrix einen oder mehrere wasserunlösliche Matrixbildner umfasst.

Entsprechend einer weiter bevorzugten Ausführungsform der erfindungsgemäßen Zusammensetzung ist es vorgesehen, dass die Matrix wasserunlöslich ist. In einer alternativen Ausführungsform der Zusammensetzung ist es vorgesehen, dass die Matrix wasserlöslich ist.

Gemäß einer weiter bevorzugten Ausführungsform der erfindungsgemäßen Zusammensetzung ist es vorgesehen, dass die Matrix als Matrixbildner eine oder mehrere Verbindungen umfasst, ausgewählt aus der Gruppe bestehend aus Celluloseestern, Celluloseethern, Poylvinylpyrrolidon/Polyvinylacetat-Mischungen, Methacrylat-Acrylat-Copolymere, Polyethylenoxide, Wachse, Fette wie zum Beispiele Glycerinester und Fettalkohole. Die genannten Substanzklassen sind als Matrixbildner für die erfindungsgemäße Zusammensetzung besonders geeignet, wobei erfindungsgemäß bevorzugt insbesondere Polyvinylacetat in Mischung mit einem Polyvinylpyrrolidon und/oder ein Glycerin-di-Behensäureester als Matrixbildner zum Einsatz kommen.

Erfindungsgemäß wird die Freisetzungsrate durch eine Anpassung des Massenverhältnis von Naloxon zu Matrixbildner reguliert. In einer Bevorzugten Ausführungsform ist das Massenverhältnis von Naloxon zu Matrixbilder 1:1, bevorzugter 1:2, noch bevorzugter 1:3, noch bevorzugter 1:4, bevorzugter 1:5, bevorzugter 1:6, noch bevorzugter 1:7, noch bevorzugter 1:8, bevorzugter 1:9 und am bevorzugtesten 1:10.

Nach einer weiter bevorzugten Ausführungsform der erfindungsgemäßen Zusammensetzung ist es vorgesehen, dass die Zusammensetzung frei von befilmten, Naloxon-haltigen Partikeln ist, wobei die Befilmung die retardierte Freisetzung des Naloxons bewirkt.

Entsprechend einer weiter bevorzugten Ausführungsform der erfindungsgemäßen Zusammensetzung ist es vorgesehen, dass die Zusammensetzung direkttablettiert ist, da diese besonders kostengünstig ist.

Nach einer weiter bevorzugten Ausführungsform der erfindungsgemäßen Zusammensetzung ist es vorgesehen, dass die Zusammensetzung in Form einer Tablette, Kapsel, eines Granulats, einer Mikrotablette, von extrudierten Partikeln oder eines zu einer Tablette verpressten Granulats vorliegt.

Gemäß einer weiter bevorzugten Ausführungsform der erfindungsgemäßen Zusammensetzung ist es vorgesehen, dass die Zusammensetzung als Einmal-täglich- (once-a-day-formulation) oder Zweimal-täglich-Formulierung (twice-a-day-formulation) ausgebildet ist.

In der erfindungsgemäßen Zusammensetzung ist als Wirkstoff Naloxon oder ein pharmazeutisch annehmbares Salz davon enthalten, wobei Naloxon-Hydrochlorid aufgrund seiner Löslichkeit und seiner Stabilität besonders bevorzugt ist.

Das Naloxon bzw. das pharmazeutisch annehmbare Salz davon ist in der erfindungsgemäßen Zusammensetzung vorzugsweise in einer Menge von 0,1 bis 500 mg enthalten, weiter bevorzugt in einer Menge von 1 mg bis 50 mg und besonders bevorzugt in einer Menge von 3 mg, 6 mg, 12 mg, 24 mg oder 48 mg.

Die vorliegende Erfindung betrifft ferner die Verwendung der erfindungsgemäßen Zusammensetzung zur Behandlung der Opioid-induzierten Obstipation.

Im Hinblick auf eine Zusammensetzung, die insbesondere für die zweimal täglich Gabe geeignet ist, betrifft die vorliegende Erfindung ferner eine feste orale pharmazeutische Zusammensetzung, umfassend als einzigen Wirkstoff Naloxon oder ein pharmazeutisch annehmbares Salz davon, wobei die Zusammensetzung den Wirkstoff retardiert freisetzt und eine in vitro-Freisetzungsrate des Wirkstoffs, gemessen unter Anwendung der Blattrührer-Methode gemäß Eur. Ph. bei 75 U/min in 500 ml 0,1 N Salzsäure bei 37 °C, von 5 % bis 50 % in 1 h, von 10 % bis 75 % in 2 h, von 20 % bis 95 % in 4 h, von 40 % bis 100 % in 8 h, von mehr als 50 % in 12 h, von mehr als 70 % in 18 h und von mehr als 80 % in 24 h aufweist.

Im Hinblick auf eine Zusammensetzung, die insbesondere für die zweimal täglich Gabe geeignet ist, betrifft die vorliegende Erfindung ferner eine feste orale pharmazeutische Zusammensetzung, umfassend als einzigen Wirkstoff Naloxon oder ein pharmazeutisch annehmbares Salz davon, wobei die Zusammensetzung den Wirkstoff retardiert freisetzt und eine in vitro-Freisetzungsrate des Wirkstoffs, gemessen unter Anwendung der Blattrührer-Methode gemäß Eur. Ph. bei 75 U/min in 500 ml 0,1 N Salzsäure bei 37 °C, von 20 % bis 50 % in 1 h, von 40 % bis 75 % in 2 h, von 60 % bis 95 % in 4 h, von 80 % bis 100 % in 8 h und von 90 % bis 100 % in 12 h aufweist.

Im Hinblick auf eine Zusammensetzung, die insbesondere für die einmal täglich Gabe geeignet ist, betrifft die vorliegende Erfindung ferner eine feste orale pharmazeutische Zusammensetzung, umfassend als einzigen Wirkstoff Naloxon oder ein pharmazeutisch annehmbares Salz davon, wobei die Zusammensetzung den Wirkstoff retardiert freisetzt und eine in vitro-Freisetzungsrate des Wirkstoffs, gemessen unter Anwendung der Blattrührer-Methode gemäß Eur. Ph. bei 75 U/min in 500 ml 0,1 N Salzsäure bei 37 °C, von 0 % bis 50 % in 1 h, von 0 % bis 75 % in 2 h, von 10 % bis 95 % in 4 h, von 35 % bis 100 % in 8 h, von 55 % bis 100 % in 12 h, von 70 % bis 100 % in 16 h und von mehr als 90 % in 24 h aufweist.

Im Hinblick auf eine Zusammensetzung, die insbesondere für die einmal täglich Gabe geeignet ist, betrifft die vorliegende Erfindung ferner eine feste orale pharmazeutische Zusammensetzung, umfassend als einzigen Wirkstoff Naloxon oder ein pharmazeutisch annehmbares Salz davon, wobei die Zusammensetzung den Wirkstoff retardiert freisetzt und eine in vitro-Freisetzungsrate des Wirkstoffs, gemessen unter Anwendung der Blattrührer-Methode gemäß Eur. Ph. bei 75 U/min in 500 ml 0,1 N Salzsäure bei 37 °C, von 0 % bis 30 % in 1 h, von 0 % bis 40 % in 2 h, von 3 % bis 55 % in 4 h, von 10 % bis 65 % in 8 h, von 20% bis 75 % in 12 h, von 30 % bis 88 % in 16 h, von 50 % bis 100 % in 24 h und von mehr als 80 % in 36 h aufweist.

Im Hinblick auf eine Zusammensetzung, die insbesondere für die einmal täglich Gabe geeignet ist, betrifft die vorliegende Erfindung ferner eine feste orale pharmazeutische Zusammensetzung, umfassend als einzigen Wirkstoff Naloxon oder ein pharmazeutisch annehmbares Salz davon, wobei die Zusammensetzung den Wirkstoff retardiert freisetzt und eine in vitro-Freisetzungsrate des Wirkstoffs, gemessen unter Anwendung der Blattrührer-Methode gemäß Eur. Ph. bei 75 U/min in 500 ml 0,1 N Salzsäure bei 37 °C, von 10 % bis 30 % in 1 h, von 17 % bis 37 % in 2 h, von 27 % bis 47 % in 4 h, von 40 % bis 60 % in 8 h, von 50 % bis 70 % in 12 h, von 60 % bis 80 % in 16 h, von 80 % bis 100 % in 24 h aufweist.

Im Sinne einer guten Patientencompliance ist es gemäß einer weiter bevorzugten Ausführungsform der erfindungsgemäßen Zusammensetzung vorgesehen, dass die Zusammensetzung, vorzugsweise eine Tablette oder eine Kapsel, eine *in vitro*-Freisetzungsrate des Wirkstoffs, gemessen unter Anwendung der Blattrührer-Methode gemäß Eur. Ph. bei 75 U/min in 500 ml 0,1 N Salzsäure bei 37 °C, von 0 % bis 75 % in 2 h, von 3 % bis 95 % in 4 h, von 20 % bis 100 % in 10 h, von 30 % bis 100 % in 16 h, von 50 % bis 100 % in 24 h und mehr als 80 % in 36 h aufweist.

Entsprechend einer weiter bevorzugten Ausführungsform der erfindungsgemäßen Zusammensetzung ist es im Hinblick auf die Bereitstellung einer Zusammensetzung, die für zumindest 12 h zur Behandlung der Opioid-induzierten Obstipation geeignet ist, vorgesehen, dass die Zusammensetzung eine in vitro-Freisetzungsrate des Wirkstoffs von 0 % bis 50 % in 2 h, von 5 % bis 95 % in 4 h, von 20 % bis 90 % in 10 h, von mehr als 70 % in 18 h und von mehr als 80 % in 24 h aufweist.

Aufgabe der vorliegenden Erfindung ist es auch eine Tablette bereitzustellen, die als Wirkstoffe einen Opioid-Agonisten oder ein pharmazeutisch annehmbares Salz davon und einen Opioid-Antagonisten oder ein pharmazeutisch annehmbares Salz davon umfasst und die zur parallelen Behandlung von Schmerz und Opioid-induzierter Obstipation geeignet ist, wobei mittels der Tablette ein individueller Behandlungserfolg leicht optimiert werden kann.

Diese Aufgabe wird erfindungsgemäß gelöst durch eine Mehrschichttablette, umfassend zumindest

- eine erste Wirkstoffschicht, die als einzigen Wirkstoff einen Opioid-Agonisten oder ein pharmazeutisch annehmbares Salz davon enthält; und
- eine zweite Wirkstoffschicht, die als einzigen Wirkstoff ein Opioid-Antagonisten oder ein pharmazeutisch annehmbares Salz davon enthält,

wobei die erste und die zweite Wirkstoffschicht den Wirkstoff retardiert freisetzen.

Die erfindungsgemäße Mehrschichttablette ist zur parallelen Behandlung von Schmerz und Opioid-induzierter Obstipation geeignet, wobei eine variable Freisetzungskinetik für beide Wirkstoffe einfach realisiert und daher ein individueller Behandlungserfolg leicht optimiert werden kann.

Die erfindungsgemäße Mehrschichttablette hat ferner den Vorteil, dass in jeder Schicht ein unterschiedliches Retardierungssysteme, z.B. mit einer Retardschicht beschichtete Pellets oder eine retardiert freisetzende Matrix, zum Einsatz kommen kann. Dadurch kann eine bestimmte Freisetzungskinetik für jeden Wirkstoff und ein variables Mengenverhältnis der Wirkstoffe individuell abgestimmt auf einen bestimmten Patienten besonders einfach realisiert werden.

Die erfindungsgemäße Mehrschichttablette hat darüber hinaus den Vorteil, dass mittels derselben viel genauere Dosierungen der beiden Wirkstoffe möglich sind. Dies kommt insbesondere bei kleinen Dosen zum Tragen.

Die erfindungsgemäße Tablette ist darüber hinaus dann von Vorteil, wenn die beiden Wirkstoffe miteinander nicht kompatibel sind.

Entsprechend einer besonders bevorzugten Ausführungsform der erfindungsgemäßen Mehrschichttablette ist es vorgesehen, dass die Mehrschichttablette eine Zweischichttablette ist. Die erfindungsgemäße Mehrschichttablette kann in Form einer Zweischichttablette verfahrenstechnisch einfach und damit kostengünstig realisiert werden.

Gemäß einer weiter bevorzugten Ausführungsform der erfindungsgemäßen Mehrschichttablette ist es vorgesehen, dass die zweite Wirkstoffschicht eine Matrix umfasst, welche die Freisetzung des Opioid-Antagonisten oder des pharmazeutisch annehmbaren Salzes davon retardiert. Eine retardierend wirkende Matrix kann verfahrenstechnisch einfach und damit kostengünstig hergestellt werden. Dabei kann es sich bei der Matrix erfindungsgemäß bevorzugt um eine sogenannte Gerüstmatrix, die quellbar oder nicht-quellbar sein kann, oder um eine sogenannte erodierende Matrix handeln. Die Matrix kann auch Eigenschaften von beiden, sowohl Gerüstmatrix als auch erodierende Matrix, aufweisen.

Bei einer Gerüstmatrix ist der Wirkstoff in einem Matrixgerüst inkorporiert. Während des Transportes der beladenen Gerüstmatrix durch den Gastrointestinaltrakt wird der Wirkstoff allmählich von den Verdauungssäften aus dem Matrixgerüst herausgelöst. Nachdem dies geschehen ist, wird das Matrixgerüst in mehr oder weniger unveränderter bzw. aufgequollener Form ausgeschieden. Bei einer erodierenden Matrix hingegen wird die Matrix abgebaut oder erodiert, wodurch Wirkstoffpartikel an die Oberfläche gelangen und sich auflösen. Dabei ist die Freisetzungsrate vom Ausmaß des Matrixabbaus bzw. deren Erosion abhängig.

Nach einer weiter bevorzugten Ausführungsform der erfindungsgemäßen Mehrschichttablette ist es vorgesehen, dass die Matrix einen oder mehrere wasserunlösliche Matrixbildner enthält. In einer alternativen Ausführungsform ist es vorgesehen, dass die MAtrix einen oder mehrere wasserlösliche Matrixbildner enthält.

Entsprechend einer weiter bevorzugten Ausführungsform der erfindungsgemäßen Mehrschichttablette ist es vorgesehen, dass die Matrix als Matrixbildner einen oder mehrere Bestandteile umfasst ausgewählt aus der Gruppe bestehend aus Celluloseestern, Celluloseethern, Poylvinylpyrrolidon/Polyvinylacetat-Mischungen, Methacrylat-Acrylat-Copolymere, Polyethylenoxide, Wachse, Fette wie Glycerinester, und Fettalkohole. Die besagten Bestandteile sind als Matrixbildner für die erfindungsgemäße Tablette besonders geeignet, wobei erfindungsgemäß bevorzugt insbesondere Polyvinylacetat in Mischung mit einem Polyvinylpyrrolidon und/oder ein Glycerin-di-Behensäureester als Matrixbildner zum Einsatz kommen.

Erfindungsgemäß wird die Freisetzungsrate durch eine Anpassung des Massenverhältnis von Naloxon zu Matrixbildner reguliert. In einer Bevorzugten Ausführungsform ist das Massenverhältnis von Naloxon zu Matrixbilder 1:1, bevorzugter 1:2, noch bevorzugter 1:3, noch bevorzugter 1:4, bevorzugter 1:5, bevorzugter 1:6, noch bevorzugter 1:7, noch bevorzugter 1:8, bevorzugter 1:9 und am bevorzugtesten 1:10.

Gemäß einer weiter bevorzugten Ausführungsform der erfindungsgemäßen Mehrschichttablette ist es vorgesehen, dass die zweite Wirkstoffschicht den Wirkstoff, d.h. den Opioid-Antagonisten oder das pharmazeutisch annehmbare Salz davon, unabhängig vom Umgebungs-pH-Wert des Magen-Darm-Traktes freisetzt. Dadurch wird gewährleistet, dass der gesamte Gastrointestinaltrakt unabhängig vom jeweiligen lokalen pH-Milieu gleichmäßig mit dem Opioid-Antagonisten bzw. einem Salz davon versorgt wird, wodurch eine weitere Optimierung des Behandlungserfolgs erzielt werden kann. Die pH-unabhängige Wirkstofffreisetzung der erfindungsgemäßen Zusammensetzung kann durch Auswahl von geeigneten Komponenten erzielt werden, wobei im Gastrointestinaltrakt lokale pH-Werte von etwa 1,2 (Magen) bis ca. 7,0 Kolon herrschen.

Nach einer weiter bevorzugten Ausführungsform der erfindungsgemäßen Mehrschichttablette ist es vorgesehen, dass der Opioid-Antagonist ausgewählt ist aus der Gruppe bestehend aus Naloxon, N-Methylnaloxon und N-Methylnaltrexon sowie pharmazeutisch annehmbaren Salzen davon, wobei Naloxon-Hydrochlorid erfindungsgemäß besonders bevorzugt ist.

Entsprechend einer weiter bevorzugten Ausführungsform der erfindungsgemäßen Mehrschichttablette ist es vorgesehen, dass der Opioid-Agonist bzw. das pharmazeutisch annehmbare Salz davon in der ersten Wirkstoffschicht in Form von Pellets enthalten ist, die den Opioid-Agonisten enthalten und auf die eine die Freisetzung des Opioid-Agonisten retardierende Schicht aufgebracht ist.

Gemäß einer weiter bevorzugten Ausführungsform der erfindungsgemäßen Mehrschichttablette ist es vorgesehen, dass Opioid-Agonist ausgewählt ist aus der Gruppe bestehend aus: Alfentanil, Allylprodin, Alphaprodin, Anileridin, Benzylmorphin, Bezitramid, Buprenorphin, Butorphanol, Clonitazen, Codein, Besomorphin, Dextromoramid, Dezocin, Diampromid, Diamorphon, Dihydrocodein, Dihydromorphin, Dimenoxadol, Dimepheptanol, Dimethylthiambuten, Dioxaphetylbutyrat, Dipipanon, Eptazocin, Ethoheptazin, Ethylmethylthiambuten, Ethylmorphin, Etonitazen, Fentanyl, Heroin, Hydrocodon, Hydromorphon, Hydroxypethidin, Isomethadon, Ketobemidon, Levorphanol, Levophenacylmorphan, Lofentanil, Meperidin, Meptazinol, Metazocin, Methadon, Metopon, Morphin, Myrophin, Narcein, Nicomorphin, Norlevorphanol, Normethadon, Nalorphin, Nalbuphen, Normorphin, Norpipanon, Opium, Oxycodon, Oxymorphon, Papaveretum, Pentazocin, Phenadoxon, Phenomorphan, Phenazocin, Phenoperidin, Piminodin, Piritramid, Propheptazin, Promedol, Properidin, Propoxyphen, Sufentanil, Tilidin, und Tramadol sowie deren pharmazeutisch annehmbare Salze, wobei Hydrocodon, Morphin, Hydromorphon, Oxycodon, Buprenorphin, Codein, Fentanyl, Levorphanol, Meperidin, Methadon, Levomethadon und Dextromethadon sowie deren pharmazeutisch annehmbare Salze erfindungsgemäß besonders bevorzugt sind.

Nach einer weiter bevorzugten Ausführungsform der erfindungsgemäßen Mehrschichttablette ist es vorgesehen, dass zumindest die zweite Wirkstoffschicht direkttablettiert ist, da diese so besonders kostengünstig herstellbar ist.

Entsprechend einer weiter bevorzugten Ausführungsform der erfindungsgemäßen Mehrschichttablette ist es vorgesehen, dass die Tablette für die Einmal-tägliche-Gabe ausgebildet ist (once-daily-formulation).

Gemäß einer weiter bevorzugten Ausführungsform der erfindungsgemäßen Mehrschichttablette ist es vorgesehen, dass die Tablette für die Zweimal-tägliche-Gabe ausgebildet ist (twice-daily-formulation).

Nach einer weiter bevorzugten Ausführungsform der erfindungsgemäßen Mehrschichttablette ist es vorgesehen, dass die Mehrschichttablette von 0,1 mg bis 500 mg des Opioid-Agonisten bzw. eines pharmazeutisch annehmbaren Salzes davon enthält und von 0,1 mg bis 500 mg des Opioid-Antagonisten bzw. eines pharmazeutisch annehmbaren Salzes davon enthält, erfindungsgemäß besonders bevorzugt in einem Verhältnis (Agonist:Antagonist) von 1:10 bis 10:1.

Die vorliegende Erfindung betrifft ferner ein erstes Verfahren zur Herstellung der erfindungsgemäßen Mehrschichttablette umfassend die Schritte

- Bereitstellen einer ersten Tablettiermasse, umfassend als einzigen Wirkstoff einen Opioid-Agonisten oder ein pharmazeutisch annehmbares Salz davon sowie ein Retardierungsmittel;
- Bereitstellen einer zweiten Tablettiermasse, umfassend als einzigen Wirkstoff einen Opioid-Antagonisten oder ein pharmazeutisch annehmbares Salz davon sowie ein Retardierungsmittel;
- Befüllen eines ersten Füllschuhs eines Tablettenpresswerkzeuges mit der ersten Tablettiermasse und eines zweiten Füllschuhs des Tablettenpresswerkzeuges mit der zweiten Tablettiermasse;
- Verpressen der ersten und zweiten Tablettiermasse unter Erhalt einer Mehrschichttablette.

Die vorliegende Erfindung betrifft ferner ein zweites Verfahren zur Herstellung der erfindungsgemäßen Mehrschichttablette umfassend die Schritte

- Bereitstellen einer ersten Tablettiermasse, umfassend als einzigen Wirkstoff einen Opioid-Agonisten oder ein pharmazeutisch annehmbares Salz davon, wobei der Opioid-Agonist oder das pharmazeutisch annehmbare Salz davon in der ersten Tablettiermasse in Form von Pellets enthalten ist, die den Opioid-Agonisten enthalten und auf die eine die Freisetzung des Opioid-Agonisten retardierende Schicht aufgebracht ist;

- Bereitstellen einer zweiten Tablettiermasse, umfassend als einzigen Wirkstoff einen Opioid-Antagonisten oder ein pharmazeutisch annehmbares Salz davon sowie ein Retardierungsmittel;
- Befüllen eines ersten Füllschuhs eines Tablettenpresswerkzeuges mit der ersten Tablettiermasse und eines zweiten Füllschuhs des Tablettenpresswerkzeuges mit der zweiten Tablettiermasse;
- Verpressen der ersten und zweiten Tablettiermasse unter Erhalt einer Mehrschichttablette.

Die vorliegende Erfindung betrifft ferner die Verwendung der erfindungsgemäßen Mehrschichttablette zur Behandlung von Schmerzen und zur gleichzeitigen Behandlung der Opioid-induzierten Obstipation.

Die nachfolgenden Ausführungsbeispiele dienen der Erläuterung der Erfindung.

**BEISPIELE**

**ORALE ZUSAMMENSETZUNG**

Die nachfolgenden Ausführungsbeispiele dienen im Zusammenhang mit der Zeichnung der Erläuterung der Erfindung. Es zeigt:

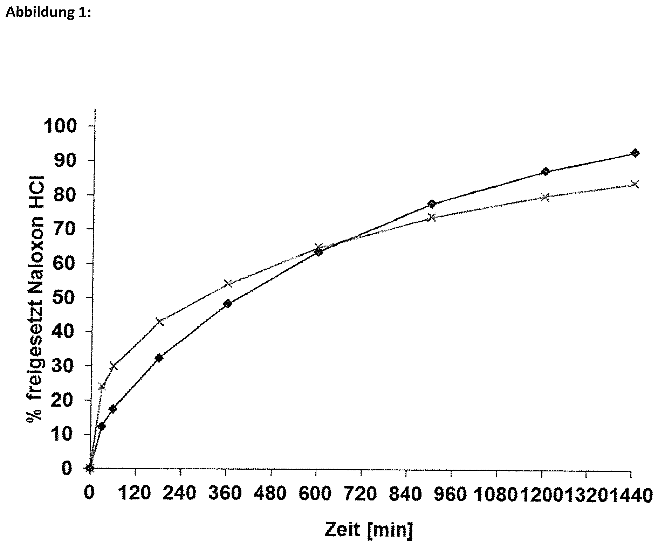

**Fig. 1: Freisetzungsprofile der Tabletten gemäß der Ausführungsbeispiele 1 und 2.**

**Ausführungsbeispiel 1:**

Es wurden Tabletten der folgenden Zusammensetzung hergestellt:

| Substanz | Funktion | Gewicht [mg] |
|---|---|---|
| Naloxon-Hydrochlorid | Wirkstoff | 48,00 |
| Glycerin-di-behensäureester | Retardierungsmittel | 204,64 |
| kolloidales Siliciumdioxid | Fließregulierungsmittel | 19,00 |
| Magnesiumstearat | Schmiermittel | 2,36 |
| | **Gesamtgewicht der Tablette** | **274,00** |

Die Bestandteile Naloxon-Hydrochlorid und Glycerin-di-behensäureester wurden gesiebt und miteinander vermischt. Zu der resultierenden Mischung wurde zunächst das kolloidale Siliciumdioxid in gesiebter Form und dann das Magnesiumstearat zugemischt. Die so erhaltene Mischung wurde mittels einer konventionellen Tablettenpresse zur Tabletten verpresst.

**Ausführungsbeispiel 2:**

Es wurden Tabletten der folgenden Zusammensetzung analog Ausführungsbeispiel 1 hergestellt:

| Substanz | Funktion | Gewicht [mg] |
|---|---|---|
| Naloxon-Hydrochlorid | Wirkstoff | 12,00 |
| Kollidon SR | Retardierungsmittel | 63,16 |
| Vivapur 200 | Füllmittel | 7,00 |
| kolloidales Siliciumdioxid | Fließregulierungsmittel | 1,24 |
| Magnesiumstearat | Schmiermittel | 0,60 |
| | **Gesamtgewicht der Tablette** | **84,00** |
| Kollidon® SR bestehend aus 80 Gew.-% Polyvinylacetat, 19 Gew.-% Povidon, 0,8 Gew.-% Na-Laurylsulfat und 0,2 Gew.-% kolloidales Siliciumdioxid. | | |

**Ausführungsbeispiel 3:**

Es wurden befilmte Zweischichttabletten der folgenden Zusammensetzung hergestellt:

| Substanz | Funktion | Gewicht [mg] |
|---|---|---|
| **Naloxon-Schicht** | | |
| Naloxon-Hydrochlorid | Wirkstoff | 3,00 |
| Kollidon® SR | Retardierungsmittel | 17,00 |
| Glycerin-di-behensäureester | Retardierungsmittel | 4,75 |
| kolloidales Siliciumdioxid | Fließregulierungsmittel | 0,60 |
| Magnesiumstearat | Schmiermittel | 0,15 |
| | **Gesamtgewicht der Naloxon-Schicht** | **25,5** |
| **Placebo-Schicht** | | |
| Zucker-Pellets (Durchmesser: 500-600 μm) | Träger | 10,00 |
| Hypromellose | Füllstoff | 10,00 |
| mikrokristalline Cellulose | Füllstoff | 10,00 |
| kolloidales Siliciumdioxid | Fließregulierungsmittel | 0,25 |
| Magnesiumstearat | Schmiermittel | 0,25 |
| | **Gesamtgewicht der Placebo-Schicht** | **30,50** |
| | **Gesamtgewicht des Zweischichttablettenkerns** | **56,00** |
| Opadry® | Tablettenfilm | 3,00 |
| | **Gesamtgewicht der Zweischichttablette** | **59,00** |

Die Bestandteile der Naloxon-Schicht, d.h. Naloxon-Hydrochlorid, Kollidon® SR, Glycerin-di-behensäureester, kolloidales Siliciumdioxid und Magnesiumstearat wurden gesiebt und miteinander zu einer ersten pulverförmigen Mischung vermischt. Ferner wurden die Bestandteile der Placebo-Schicht, d.h. Zucker-Pellets, Hypromellose, mikrokristalline Cellulose, kolloidales Siliciumdioxid und Magnesiumstearat gesiebt und miteinander zu einer zweiten pulverförmigen Mischung vermischt.

Die erste und die zweite Mischung wurden mittels einer konventionellen Zweischichttablettenpresse zur Zweischichttablettenkernen verpresst. Die so erhaltenen Zweischichttablettenkerne wurden in einem Coater mittels Opadry® gelöst in Wasser bei einer Temperatur von 30 °C bis 50 °C befilmt unter Erhalt von Zweischichttabletten.

**Freisetzungsprofile**

Die in vitro Freisetzungsprofile der Tabletten gemäß der Ausführungsbeispiele 1 und 2 wurden unter Anwendung der Blattrührer-Apparatur (Apparatur 2) und der Blattrührer-Methode gemäß Eur.Ph. (Europäisches Arzneibuch, 7. Ausgabe, 3. Nachtrag, 2.9.3 "Wirkstofffreisetzung aus festen Arzneiformen", S. 5519-5526) bei 75 U/min in 500 ml 0,1 N Salzsäure bei 37 °C bestimmt. Die Menge an freigesetztem Wirkstoff wurde mittels UV-Detektion bei 220 nm bestimmt.

Die in vitro Freisetzungsprofile der Tabletten gemäß der Ausführungsbeispiele 1 (♦) und 2 (x) sind in der Figur 1 dargestellt.

**MEHRSCHICHTTABLETTE**

**Ausführungsbeispiel 1:**

Es wurden befilmte Doppelschichttabletten der folgenden Zusammensetzung hergestellt:

| Substanz | Funktion | Gewicht [mg] |
|---|---|---|
| **Oxycodon-Schicht** | | |
| retardierte Oxycodon-Pellets (enthalten 3 mg Oxycodon HCl) | Wirkstoff | 10,00 |
| mikrokristalline Cellulose | Füllstoff | 24,50 |
| kolloidales Siliciumdioxid | Fließregulierungsmittel | 0,25 |
| Magnesiumstearat | Schmiermittel | 0,25 |
| | **Gesamtgewicht der Oxycodon-Schicht** | **35,00** |
| | | |
| **Naloxon-Schicht** | | |
| Naloxon-Hydrochlorid | Wirkstoff | 3,00 |
| Kollidon® SR | Retardierungsmittel | 21,75 |
| kolloidales Siliciumdioxid | Fließregulierungsmittel | 0,50 |
| Magnesiumstearat | Schmiermittel | 0,25 |
| | **Gesamtgewicht der Naloxon-Schicht** | **25,50** |
| | **Gesamtgewicht des Zweischichttablettenkerns** | **60,50** |
| Opadry II® | Tablettenfilm | 2,50 |
| | **Gesamtgewicht der befilmten Tablette** | **63,00** |

Die Bestandteile der Oxycodon-Schicht, d.h. die retardierten Oxycodon-Pellets, mikrokristalline Cellulose, kolloidales Siliciumdioxid und Magnesiumstearat wurden gesiebt und miteinander zu einer ersten Mischung vermischt.

Ferner wurden die Bestandteile der Naloxon-Schicht, d.h. Naloxon-Hydrochlorid, Kollidon® SR, kolloidales Siliciumdioxid und Magnesiumstearat gesiebt und miteinander zu einer zweiten pulverförmigen Mischung vermischt.

Die erste und die zweite Mischung wurden mittels einer konventionellen Zweischichttablettenpresse zu Zweischichttablettenkernen verpresst. Die so erhaltenen Zweischichttablettenkerne wurden in einem Coater mittels Opadry II® gelöst in Wasser bei einer Temperatur von 30 °C bis 50 °C befilmt.

Die retardierten Oxycodon-Pellets wiesen die nachstehende Zusammensetzung auf und wurden wie im Stand der Technik bekannt hergestellt.

| Substanz | Funktion | Gewicht [mg] |
|---|---|---|
| **Oxycodon Pellets** | | |
| Oxycodon Hydrochlorid | Wirkstoff | 3,00 |
| Pellets neutral | Träger | 2,50 |
| Povidon | Bindemittel | 0,50 |
| **retardierende Schicht** | | |
| Ethylcellulose | Retardierungsmittel | 3,00 |
| Hydroxypropylcellulose | | 0,50 |
| Triethylcitrate | | 0,50 |
| | **Gesamtgewicht der retardierten Oxycodon Pellets** | 10,00 |

Kollidon® SR bestehend aus 80 Gew.-% Polyvinylacetat, 19 Gew.-% Povidon, 0,8 Gew.-% Na-Laurylsulfat und 0,2 Gew.-% kolloidales Siliciumdioxid.

Opadry II® besteht aus Polyvinylalkohol, Eisenoxid oder Titandioxid, Macrogol und Talkum.

**Ausführungsbeispiel 2:**

Es wurden befilmte Doppelschichttabletten der folgenden Zusammensetzung analog Ausführungsbeispiel 1 hergestellt:

| Substanz | Funktion | Gewicht [mg] |
|---|---|---|
| **Oxycodon-Schicht** | | |
| retardierte Oxycodon-Pellets (enthalten 9 mg Oxycodon HCl) | Wirkstoff | 30,00 |
| mikrokristalline Cellulose | Füllstoff | 30,00 |
| kolloidales Siliciumdioxid | Fließregulierungsmittel | 1,00 |
| Magnesiumstearat | Schmiermittel | 1,00 |
| | **Gesamtgewicht der Oxycodon-Schicht** | **62,00** |
| | | |
| **Naloxon-Schicht** | | |
| Naloxon-Hydrochlorid | Wirkstoff | 6,00 |
| Glycerin-di-behensäureester | Retardierungsmittel | 23,00 |
| Mikrokristalline Cellulose | Füllstoff | 20,00 |
| kolloidales Siliciumdioxid | Fließregulierungsmittel | 1,50 |
| Magnesiumstearat | Schmiermittel | 0,50 |
| | **Gesamtgewicht der Naloxon-Schicht** | **51,00** |
| | **Gesamtgewicht des Zweischichttablettenkerns** | **113,00** |
| Opadry II® | Tablettenfilm | 8,00 |
| | **Gesamtgewicht der befilmten Tablette** | **121,00** |

**Ausführungsbeispiel 3:**

Es wurden befilmte Doppelschichttabletten der folgenden Zusammensetzung analog Ausführungsbeispiel 1 hergestellt:

| Substanz | Funktion | Gewicht [mg] |
|---|---|---|
| **Oxycodon-Schicht** | | |
| retardierte Oxycodon-Pellets (enthalten 24 mg Oxycodon HCl) | Wirkstoff | 80,00 |
| mikrokristalline Cellulose | Füllstoff | 242,00 |
| kolloidales Siliciumdioxid | Fließregulierungsmittel | 4,00 |
| Magnesiumstearat | Schmiermittel | 4,00 |
| | **Gesamtgewicht der Oxycodon-Schicht** | **330,00** |
| | | |

(fortgesetzt)

| Naloxon-Schicht | | |
|---|---|---|
| Naloxon-Hydrochlorid | Wirkstoff | 48,00 |
| mikrokristalline Cellulose | Füllstoff | 84,00 |
| Kollidon® SR | Retardierungsmittel | 204,00 |
| kolloidales Siliciumdioxid | Fließregulierungsmittel | 10,00 |
| Magnesiumstearat | Schmiermittel | 2,00 |
| | **Gesamtgewicht der Naloxon-Schicht** | **348,00** |
| | **Gesamtgewicht des Zweischichttablettenkerns** | **678,00** |
| Opadry® | Tablettenfilm | 22,00 |
| | **Gesamtgewicht der befilmten Tablette** | **700,00** |

## Abbildungsverzeichnis

**Abbildung 1:** Freisetzungsprofil von Naloxol aus einer erfindungsgemäßen Zusammensetzung; (x) Ausführungsbeispiel 1, (♦) Ausführungsbeispiel 2.

## Patentansprüche

1. Feste orale pharmazeutische Zusammensetzung, umfassend als einzigen Wirkstoff Naloxon oder ein pharmazeutisch annehmbares Salz davon, wobei die Zusammensetzung den Wirkstoff retardiert freisetzt und eine in vitro-Freisetzungsrate des Wirkstoffs, gemessen unter Anwendung der Blattrührer-Methode gemäß Eur. Ph. bei 75 U/min in 500 ml 0,1 N Salzsäure bei 37 °C, von 0 % bis 75 % in 2 h, von 3 % bis 95 % in 4 h, von 20 % bis 100 % in 10 h, von 30 % bis 100 % in 16 h, von 50 % bis 100 % in 24 h und mehr als 80 % in 36 h aufweist, wobei die Zusammensetzung eine Matrix umfasst, wobei der Matrixbildner Glycerin-di-Behensäureester umfasst.

2. Zusammensetzung nach Anspruch 1, wobei die Zusammensetzung den Wirkstoff unabhängig vom Umgebungs-pH-Wert des Magen-Darm-Traktes freisetzt.

3. Zusammensetzung nach Anspruch 1 oder 2, **dadurch gekennzeichnet, dass** die Zusammensetzung eine in vitro-Freisetzungsrate des Wirkstoffs von 0 % bis 50 % in 2 h, von 5 % bis 95 % in 4 h, von 20 % bis 90 % in 10 h, von mehr als 70 % in 18 h und von mehr als 80 % in 24 h aufweist.

4. Zusammensetzung nach einem der voranstehenden Ansprüche, **dadurch gekennzeichnet, dass** die Zusammensetzung eine in vitro-Freisetzungsrate des Wirkstoffs von 0 % bis 38 % in 2 h, von 5 % bis 55 % in 4 h und von 20 % bis 75 % in 10 h aufweist.

5. Zusammensetzung nach Anspruch 1 oder 2, **dadurch gekennzeichnet, dass** die Zusammensetzung eine in vitro-Freisetzungsrate des Wirkstoffs von 0 % bis 50 % in 1 h, von 10 % bis 95 % in 4 h, von 35 % bis 100 % in 8 h, von 55 % bis 100 % in 12 h, von 70 % bis 100 % in 16 h und von mehr als 90 % in 24 h aufweist.

6. Zusammensetzung nach Anspruch 5, **dadurch gekennzeichnet, dass** die Zusammensetzung eine in vitro-Freisetzungsrate des Wirkstoffs von 0 % bis 30 % in 1 h, von 0 % bis 40 % in 2 h, von 3 % bis 55 % in 4 h, von 10 % bis 65 % in 8 h, von 20 % bis 75 % in 12 h, von 30 % bis 88 % in 16 h, von 50 % bis 100 % in 24 h und von mehr als 80 % in 36 h aufweist.

7. Zusammensetzung nach einem der Ansprüche 1 bis 6, **dadurch gekennzeichnet, dass** eine Matrix den Wirkstoff retardiert freisetzt.

8. Zusammensetzung nach Anspruch 7, wobei die Matrix als Matrixbildner eine oder mehrere Verbindungen umfasst, ausgewählt aus der Gruppe bestehend aus Celluloseestern, Celluloseethern, Poylvinylpyrrolidon/Polyvinylacetat-Mischungen, Methacrylat-Acrylat-Copolymere, Wachse, Fette wie Glycerinester, und Fettalkohole.

9. Zusammensetzung nach einem der Ansprüche 7 oder 8, **dadurch gekennzeichnet, dass** die Matrix als Matrixbildner eine Poylvinylpyrrolidon/Polyvinylacetat-Mischung umfasst.

10. Zusammensetzung nach einem der Ansprüche 7 bis 9, **dadurch gekennzeichnet, dass** die Matrix als Matrixbildner Glycerin-di-Behensäureester umfasst.

11. Zusammensetzung nach einem der voranstehenden Ansprüche, **dadurch gekennzeichnet, dass** die Zusammensetzung direkttablettiert ist.

12. Zusammensetzung nach einem der voranstehenden Ansprüche, **dadurch gekennzeichnet, dass** die Zusammensetzung als Einmal-täglich-Formulierung ausgebildet ist.

13. Zusammensetzung nach einem der voranstehenden Ansprüche 1 bis 11, **dadurch gekennzeichnet, dass** die Zusammensetzung als Zweimal-täglich-Formulierung ausgebildet ist.

14. Feste orale pharmazeutische Zusammensetzung nach einem der Ansprüche 1 bis 13 zur Verwendung in der Behandlung der Opioid-induzierten Obstipation.


**Claims**

1. Solid oral pharmaceutical composition comprising as sole active ingredient naloxone or a pharmaceutically acceptable salt thereof, wherein the composition exhibits delayed release of the active ingredient and an in-vitro dissolution of the active ingredient, measured at 75 rpm in 500 ml of 0.1 N hydrochloric acid at 37°C using the Ph. Eur. paddle stirrer method, of from 0% to 75% in 2 h, from 3% to 95% in 4 h, from 20% to 100% in 10 h, from 30% to 100% in 16 h, from 50% to 100% in 24 h and over 80% in 36 h, the composition comprising a matrix wherein the matrix-forming agent comprises glycerol dibehenate.

2. Composition according to Claim 1, wherein the release of the active ingredient by the composition is independent of the ambient pH in the gastrointestinal tract.

3. Composition according to Claim 1 or 2, **characterized in that** the composition exhibits an in-vitro dissolution of the active ingredient of from 0% to 50% in 2 h, from 5% to 95% in 4 h, from 20% to 90% in 10 h, of more than 70% in 18 h and of more than 80% in 24 h.

4. Composition according to any of the preceding claims, **characterized in that** the composition exhibits an in-vitro dissolution of the active ingredient of from 0% to 38% in 2 h, from 5% to 55% in 4 h and from 20% to 75% in 10 h.

5. Composition according to Claim 1 or 2, **characterized in that** the composition exhibits an in-vitro dissolution of the active ingredient of from 0% to 50% in 1 h, from 10% to 95% in 4 h, from 35% to 100% in 8 h, from 55% to 100% in 12 h, from 70% to 100% in 16 h and of more than 90% in 24 h.

6. Composition according to Claim 5, **characterized in that** the composition exhibits an in-vitro dissolution of the active ingredient of from 0% to 30% in 1 h, from 0% to 40% in 2 h, from 3% to 55% in 4 h, from 10% to 65% in 8 h, from 20% to 75% in 12 h, from 30% to 88% in 16 h, from 50% to 100% in 24 h and of more than 80% in 36 h.

7. Composition according to any of Claims 1 to 6, **characterized in that** a matrix exhibits delayed release of the active ingredient.

8. Composition according to Claim 7, **characterized in that** the matrix comprises as matrix-forming agent one or more compounds selected from the group consisting of cellulose esters, cellulose ethers, polyvinylpyrrolidone/polyvinyl acetate mixtures, methacrylate-acrylate copolymers, waxes, fats such as glycerol esters and fatty alcohols.

9. Composition according to either of Claims 7 and 8, **characterized in that** the matrix comprises as matrix-forming agent a polyvinylpyrrolidone/ polyvinyl acetate mixture.

**10.** Composition according to any of Claims 7 to 9, **characterized in that** the matrix comprises as matrix-forming agent glycerol dibehenate.

**11.** Composition according to any of the preceding claims, **characterized in that** the composition undergoes direct tableting.

**12.** Composition according to any of the preceding claims, **characterized in that** the composition is formed as a once-daily formulation.

**13.** Composition according to any of the preceding Claims 1-11, **characterized in that** the composition is formed as a twice-daily formulation.

**14.** Solid oral pharmaceutical formulation according to any of Claims 1 to 13 for use in the treatment of opioid-induced constipation.


**Revendications**

**1.** Composition pharmaceutique orale solide, comprenant, en tant qu'unique principe actif, de la naloxone ou un sel pharmaceutiquement acceptable correspondant, la composition libérant le principe actif de manière retardée et présentant un taux de libération *in vitro* du principe actif, mesuré par l'application de la méthode à agitateur à pales selon la Ph. Eur. à 75 t/min dans 500 ml d'acide chlorhydrique 0,1 N à 37 °C, de 0 % à 75 % en 2 h, de 3 % à 95 % en 4 h, de 20 % à 100 % en 10 h, de 30 % à 100 % en 16 h, de 50 % à 100 % en 24 h et de plus de 80 % en 36 h, la composition comprenant une matrice, l'agent de formation de matrice comprenant un diester avec l'acide béhénylique de la glycérine.

**2.** Composition selon la revendication 1, la composition libérant le principe actif indépendamment de la valeur de pH environnante du tract gastro-intestinal.

**3.** Composition selon la revendication 1 ou 2, **caractérisée en ce que** la composition présente un taux de libération *in vitro* du principe actif de 0 % à 50 % en 2 h, de 5 % à 95 % en 4 h, de 20 % à 90 % en 10 h, de plus de 70 % en 18 h et de plus de 80 % en 24 h.

**4.** Composition selon l'une quelconque des revendications précédentes, **caractérisée en ce que** la composition présente un taux de libération *in vitro* du principe actif de 0 % à 38 % en 2 h, de 5 % à 55 % en 4 h et de 20 % à 75 % en 10 h.

**5.** Composition selon la revendication 1 ou 2, **caractérisée en ce que** la composition présente un taux de libération *in vitro* du principe actif de 0 % à 50 % en 1 h, de 10 % à 95 % en 4 h, de 35 % à 100 % en 8 h, de 55 % à 100 % en 12 h, de 70 % à 100 % en 16 h et de plus de 90 % en 24 h.

**6.** Composition selon la revendication 5, **caractérisée en ce que** la composition présente un taux de libération *in vitro* du principe actif de 0 % à 30 % en 1 h, de 0 % à 40 % en 2 h, de 3 % à 55 % en 4 h, de 10 % à 65 % en 8 h, de 20 % à 75 % en 12 h, de 30 % à 88 % en 16 h, de 50 % à 100 % en 24 h et de plus de 80 % en 36 h.

**7.** Composition selon l'une quelconque des revendications 1 à 6, **caractérisée en ce qu'**une matrice libère le principe actif de manière retardée.

**8.** Composition selon la revendication 7, la matrice comprenant un ou plusieurs composés en tant qu'agent de formation de matrice, choisis dans le groupe constitué par des esters de cellulose, des éthers de cellulose, des mélanges de polyvinylpyrrolidone/poly(acétate de vinyle), des copolymères de méthacrylate-acrylate, des cires, des graisses comme des esters de glycérine, et des alcools gras.

**9.** Composition selon l'une quelconque des revendications 7 et 8, **caractérisée en ce que** la matrice comprend un mélange de polyvinylpyrrolidone/poly(acétate de vinyle) en tant qu'agent de formation de matrice.

**10.** Composition selon l'une quelconque des revendications 7 à 9, **caractérisée en ce que** la matrice comprend un diester avec l'acide béhénylique de la glycérine en tant qu'agent de formation de matrice.

**11.** Composition selon l'une quelconque des revendications précédentes, **caractérisée en ce que** la composition est directement comprimée.

**12.** Composition selon l'une quelconque des revendications précédentes, **caractérisée en ce que** la composition est conçue comme formulation pour prise une fois par jour.

**13.** Composition selon l'une quelconque des revendications 1 à 11, **caractérisée en ce que** la composition est conçue comme formulation pour prise deux fois par jour.

**14.** Composition pharmaceutique orale solide selon l'une quelconque des revendications 1 à 13 pour une utilisation dans le traitement de la constipation induite par un opioïde.

...

**Abbildung 1:**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011117306 A

- WO 9825613 A2

**In der Beschreibung aufgeführte Nicht-Patentliteratur**

- Wirkstofffreisetzung aus festen Arzneiformen. Europäisches Arzneibuch. 5519-5526

- **SHAH V.P. ; TSONG Y. ; SATHE P. ; LIU J.P.** In vitro dissolution profile comparison-statistics and analysis of the similarity factor, f2. *Pharmaceutical Research,* 1998, vol. 15, 889-896
- Pufferlösungen. Europäisches Arzneibuch. 7671-7679